# EUROPÄISCHE PATENTSCHRIFT

(11) **EP 1 194 426 B1**
(45) Veröffentlichungstag und Bekanntmachung des Hinweises auf die Patenterteilung: **18.08.2004**
(21) Anmeldenummer: 00949229.9
(22) Anmeldetag: 29.06.2000
(51) Int. Cl.: C07D 405/06, C07D 307/85, C07D 307/80, C07D 413/06, A61K 31/343, A61K 31/4025, A61K 31/497, A61K 31/502, A61K 31/536, A61P 25/00

(54) **BENZOFURANDERIVATE**
BENZOFURANE DERIVATIVES
DERIVES DE BENZOFURAN

(30) Priorität: 10.07.1999 DE 19932314
(43) Veröffentlichungstag der Anmeldung: 10.04.2002
(73) Patentinhaber: MERCK PATENT GmbH, 64293 Darmstadt (DE)
(72) Erfinder: BATHE, Andreas, D-64283 Darmstadt (DE); HELFERT, Bernd, D-64372 Ober-Ramstadt (DE); BÖTTCHER, Henning, D-64287 Darmstadt (DE)
(86) Internationale Anmeldenummer: PCT/EP2000/006089
(87) Internationale Veröffentlichungsnummer: WO 2001/004112

(56) Entgegenhaltungen:
- EP-A- 0 648 767
- EP-A- 0 738 722
- WO-A-79/00426
- WO-A-94/29290
- DAUZONNE D ET AL: "Synthesis and some CNS activities of new benzofuranylacryloylpiperazines" EUROPEAN JOURNAL OF MEDICINAL CHEMISTRY.CHIMICA THERAPEUTICA,FR,EDITIONS SCIENTIFIQUE ELSEVIER, PARIS, Bd. 30, Nr. 1, 1995, Seiten 53-59, XP004040119 ISSN: 0223-5234

## Beschreibung

Die Erfindung betrifft Benzofuranderivate der Formel I 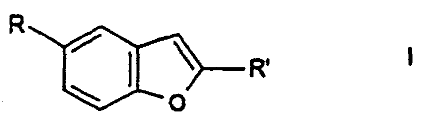 worin
- R: 1-Piperazinyl, 4-R'-Piperazinyl oder L,
- R': 2-R²-5-R³-Pyrrol-1-yl-carbonyl, 4-R⁴-Piperazin-1-yl-carbonyl, N,N-Di-(tert.-Butyloxycarbonyl)-aminocarbonyl, 1,4-Dihydro-benzo[*d*][1,2]oxazin-3-yl-carbonyl oder 3,4-Dihydro-benzo-1*H*-phthalazin-2-yl-carbonyl,
- L: Cl, Br, I oder eine freie OH-Gruppe oder ein aktivierter Ester, ein Imidazolid oder Alklylsulfonyloxy mit 1 bis 6 C-Atomen oder Arylsulfonyloxy mit 6 bis 10 C-Atomen,
- R¹, R⁴: jeweils unabhängig voneinander H, Benzyl oder eine andere Aminoschutzgruppe,
- R², R³: jeweils unabhängig voneinander H oder Alkyl mit 1-6 C-Atomen,
- R⁵, R⁶: jeweils unabhängig voneinander Alkyl mit 1-6 C-Atomen,
- Hal: F, Cl, Br oder I
bedeuten,
sowie deren Salze.

Ähnliche Verbindungen sind aus DE 43 33 254 und DE 195 14 567 bekannt.

Der Erfindung lag die Aufgabe zugrunde, neue Verbindungen zu finden, die insbesondere als Zwischenprodukte bei der Synthese von Arzneimitteln verwendet werden können, aber auch direkt zur Herstellung von Arzneimitteln Verwendung finden können.

Es wurde gefunden, daß die Verbindungen der Formel I und ihre Salze wichtige Zwischenprodukte zur Herstellung von Arzneimitteln darstellen und zugleich pharmakologische Eigenschaften besitzen. So zeigen sie beispielsweise Wirkungen auf das Zentralnervensystem.

Gegenstand der Erfindung sind die Benzofuranderivate der Formel I und ihre Salze.

Vor- und nachstehend haben die Reste R¹, R², R³, R⁴, R⁵, R⁶, R, R', L, Q und Q' die bei den Formeln I und III angegebenen Bedeutungen, falls nicht ausdrücklich etwas anderes angegeben ist.
In den vorstehenden Formeln hat A 1 bis 4, vorzugsweise 1, 2, oder 3 C-Atome. A bedeutet vorzugsweise Methyl oder Ethyl, weiterhin Propyl, Isopropyl, femer auch Butyl, Isobutyl, sek.-Butyl oder tert.-Butyl.
Der Rest Ph bedeutet Phenyl.

In den Verbindungen der Formel I, IV und V bedeuten L, Q bzw. Q' vorzugsweise Cl, Br, I oder eine reaktionsfähig abgewandelte OH-Gruppe wie z.B. ein aktivierter Ester, ein Imidazolid oder Alkylsulfonyloxy mit 1-6 C-Atomen (bevorzugt Methylsulfonyloxy) oder Arylsulfonyloxy mit 6-10 C-Atomen (bevorzugt Phenyl- oder p-Tolylsulfonyloxy).

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfembar sind, nachdem die gewünschte chemische Reaktion an anderen Stellen des Moleküls durchgeführt worden ist. Typisch für solche Gruppen sind insbesondere unsubstituierte Acyl-, Aryl-, Aralkoxymethyl- oder Aralkylgruppen. Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren und den vorliegenden Verbindungen in weitestem Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie Phenoxyacetyl; Alkoxycarbonyl wie Methoxycarbonyl, Ethoxycarbonyl, 2,2,2-Trichlorethoxycarbonyl, BOC (tert.-Butoxycarbonyl), 2-Iodethoxycarbonyl; Aralkyloxycarbonyl wie CBZ (Carbobenzoxycarbonyl, auch "Z" genannt), 4-Methoxybenzyloxycarbonyl, FMOC (9-Fluorenylmethoxycarbonyl); Arylsulfonyl wie Mtr (4-Methoxy-2,3,6-trimethylphenyl-sulfonyl). Bevorzugte Aminoschutzgruppen sind BOC und Mtr, femer CBZ oder FMOC.

Die Verbindungen der Formel I können ein oder mehrere chirale Zentren besitzen und daher in verschiedenen stereoisomeren Formen vorkommen. Die Formel I umschließt alle diese Formen.

WO 79 00426 beschreibt lediglich Methoxybenzofurane, die nicht Gegenstand der vorliegenden Anmeldung sind.

Dauzonne d et al: ,Synthesis and some CNS activities of new benzofuranylacryloylpiperazines' European jounal of medicinal chemistry. Chimica therapeutica, Fr, editions scientifique elsevier, Paris, Bd. 30, Nr. 1, 1995, Seiten 53-59 und WO 94 29290 offenbaren zwar Benzofurane, jedoch unterscheiden sich die erfindungsgemäßen Verbindungen u.a. durch den Rest R, der 1-Piperazinyl, 4-R¹-Piperazinyl oder L bedeutet.

EP-A-0 648 767 beschreibt nur solche Benzofuran-Verbindungen, die zusätzlich über einen Piperdin-Ring an einen Indolylrest geknüpft sind. Derartige Verbindungen sind nicht Gegenstand der vorliegenden Anmeldung.

EP-A-0 738 722 offenbart lediglich durch Imidazol-, Triazol- oder Tetrazol-yl-carbonyl-Reste-substituierte Benzofurane, die nicht im Anspruchsumfang der vorliegenden Anmeldung enthalten sind.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl. Methoden der organischen Chemie, Georg-Thieme Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Tetrahydrofuran (THF) oder Dioxan; Glykolether wie Ethylenglykolmonomethyl- oder -monoethytether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Nitroverbindungen wie Nitromethan oder Nitrobenzol; Ester wie Ethylacetat, gegebenenfalls auch Gemische der genannten Lösungsmittel untereinander oder Gemische mit Wasser.

Der Zusatz eines säurebindenden Mittels, beispielsweise eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums oder Calciums, oder der Zusatz einer organischen Base wie Triethylamin, Dimethylamin, Pyridin oder Chinolin oder eines Überschusses der Aminkomponente kann günstig sein. Die Reaktionszeit liegt je nach den angewendeten Bedingungen zwischen einigen Minuten und 14 Tagen, die Reaktionstemperatur zwischen 0 und 150°, normalerweise zwischen 20 und 130°.

In den Verbindungen der Formel V bedeutet der Rest Q' vorzugsweise Cl oder Br; er kann jedoch auch I, OH oder eine reaktionsfähig abgewandelte OH-Gruppe wie Alkylsulfonyloxy mit 1-6 C-Atomen (bevorzugt Methylsulfonyloxy) oder Arylsulfonyloxy mit 6-10 C-Atomen (bevorzugt Phenyl-, p-Tolylsulfonyloxy, 1- oder 2-Naphthalinsulfonyloxy) bedeuten.

In den Verbindungen der Formel IV bedeutet der Rest R vorzugsweise Br oder 4-Benrylpiperazinyl.

Die Umsetzung der Verbindungen der Formel IV mit Verbindungen der Formel V verläuft nach Methoden, wie sie für die Alkylierung von Phenolen aus der Literatur bekannt sind.

Die Verbindungen der Formel II sind zum Teil bekannt; die nicht bekannten Verbindungen können leicht analog zu den bekannten Verbindungen hergestellt werden. Die Cyclisierung erfolgt nach allgemeinen bekannten Methoden.

Die Abspaltung einer Aminoschutzgruppe aus einer Verbindung der Formel I erfolgt - je nach der benutzten Schutzgruppe - z.B. mit starken Säuren, zweckmäßig mit TFA (Trifluoressigsäure) oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich. Als inerte Lösungsmittel eignen sich vorzugsweise organische, beispielsweise Carbonsäuren wie Essigsäure, Ether wie Tetrahydrofuran oder Dioxan, Amide wie Dimethylformamid, halogenierte Kohlenwasserstoffe wie Dichlormethan, femer auch Alkohole wie Methanol, Ethanol oder Isopropanol sowie Wasser. Ferner kommen Gemische der vorgenannten Lösungsmittel in Frage. TFA wird vorzugsweise im Überschuß ohne Zusatz eines weiteren Lösungsmittels verwendet, Perchtorsäure in Form eines Gemisches aus Essigsäure und 70 %iger Perchlorsäure im Verhältnis 9 : 1. Die Reaktionstemperaturen liegen zweckmäßig zwischen etwa 0 und etwa 50°, vorzugsweise arbeitet man zwischen 15 und 30°.

Die Gruppe BOC wird bevorzugt mit TFA in Dichlormethan oder mit etwa 3 bis 5 n Salzsäure in Dioxan bei 15-30° abgespalten.

Hydrogenolytisch entfernbare Schutzgruppen (z.B. CBZ oder Benzyl) können z.B. durch Behandeln mit Wasserstoff in Gegenwart eines Katalysators (z.B. eines Edelmetallkatalysators wie Palladium, zweckmäßig auf einem Träger wie Kohle) abgespalten werden. Als Lösungsmittel eignen sich dabei die oben angegebenen, insbesondere z.B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt.

Verbindungen der Formel I in denen R' N,N-Di-(tert.-Butyloxycarbonyl)-aminocarbonyl bedeutet erhält man vorzugsweise durch Umsetzung der ungeschützten Aminocarbonylverbindung, worin
- R: 4-R¹-Piperazinyl oder L,
- L: die in Anspruch 1 angegebene Bedeutung und
- R¹: Benzyl oder eine andere Aminoschutzgruppe
bedeutet,
mit (BOC)₂O in einem inerten Lösungsmittel, wie z.B. THF oder Dioxan unter Zusatz einer Base, wie z.B. Diethylamin und vorzugsweise einer katalytischen Menge von Dimethylaminopyridin.

Eine Base der Formel I kann mit einer Säure in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, femer organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und Disulfonsäuren, Laurylschwefelsäure. Salze mit physiologisch nicht unbedenklichen Säuren, z.B. Pikrate, können zur Isolierung und /oder Aufreinigung der Verbindungen der Formel I verwendet werden.

Andererseits können Verbindungen der Formel I mit Basen (z.B. Natriumoder Kaliumhydroxid oder -carbonat) in die entsprechenden Metall-, insbesondere Alkalimetall- oder Erdalkalimetall-, oder in die entsprechenden Ammoniumsalze umgewandelt werden.

Gegenstand der Erfindung ist weiterhin die Verwendung der Verbindungen der Formel I als Zwischenprodukte zur Synthese von Arzneimitteln. Entsprechende Arzneimittel sind beispielsweise in DE 4333254 beschrieben.

Gegenstand der Erfindung ist insbesondere die Verwendung der Verbindungen der Formel I als Zwischenprodukte zur Synthese von Arzneimitteln, die Wirkungen auf das Zentralnervensystem zeigen. Ganz besonders bevorzugt ist dabei 1-[4-(5-Cyan-indol-3-yl)-Butyl]-4-(2-carbamoyl-benzofuran-5-yl)-piperazin und dessen Salze zu nennen.

Gegenstand der Erfindung ist dementsprechend insbesondere die Verwendung der Verbindungen der Formel I nach Anspruch 1
worin
- R: Cl, Br, I oder 4-R¹-Piperazinyl,
- R': 2-R²-5-R³-Pyrrol-1-yl-carbonyl, 4-R⁴-Piperazin-1-yl-carbonyl, 1,4-Dihydro-benzo[*d*][1,2]oxazin-3-yl-carbonyl oder 3,4-Dihydro-benzo-1*H*-phthalazin-2-yl-carbonyl,
- R¹: Benzyl oder eine andere Aminoschutzgruppe,
- R⁴: H, Benzyl oder eine andere Aminoschutzgruppe.
- R², R³: jeweils unabhängig voneinander H oder Alkyl mit 1-6 C-Atomen, bedeuten,
bei der Synthese von
1-[4-(5-Cyan-indol-3-yl)-Butyl]-4-(2-carbamoyl-benzofuran-5-yl)-piperazin und dessen Salzen, dadurch gekennzeichnet,
daß man 3-R-6-hydroxy-benzaldehyd,
worin R Cl, Br oder I bedeutet,
mit einer Verbindung der Formel II

X-CH₂-CO-Q II

worin
- X: Cl, Br, I oder eine freie oder funktionell abgewandelte OH-Gruppe,
- Q: OH oder OR" und
- R": Alkyl mit 1-6 C-Atomen,
bedeutet,
zu einer Verbindung der Formel IV 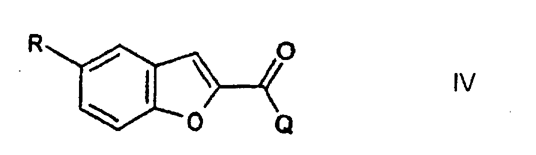 worin
- R: Cl, Br oder I,
- und Q: die angegebenen Bedeutungen bedeutet,
umsetzt,
daß man in der so erhaltenen Verbindung Q in Cl, Br, I oder eine funktionell abgewandelte OH-Gruppe umwandelt, daß man die so erhaltene Verbindung mit einer Verbindung der Formel III

R'-H III

worin
- R': 2-R²-5-R³-Pyrrol-1-yl-carbonyl, 4-R⁴-Piperazin-1-yl-carbonyl, 1,4-Dihydro-benzo[*d*][1,2]oxazin-3-yl-carbonyl oder 3,4-Dihydro-benzo-1*H*-phthalazin-2-yl-carbonyl
bedeutet,
und R², R³ und R⁴ die angegebenen Bedeutungen haben,
zu einer Verbindung der Formel I
worin
- R: Cl, Br oder I,
- R': 2-R²-5-R³-Pyrrol-1-yl-carbonyl, 4-R⁴-Piperazin-1-yl-carbonyl, 1,4-Dihydro-benzo[*d*][1,2]oxazin-3-yl-carbonyl oder 3,4-Dihydro-benzo-1*H*-phthalazin-2-yl-carbonyl,
- R⁴: H, Benzyl oder eine andere Aminoschutzgruppe,
- R², R³: jeweils unabhängig voneinander H oder Alkyl mit 1-6 C-Atomen, bedeuten,
umsetzt,
daß man in der so erhaltenen Verbindung der Formel I den Rest R in einen anderen Rest R umwandelt,
indem man Übergangsmetall-katalysiert mit einer Verbindung der Formel V

4-R¹-Piperazin V

worin
- R¹: Benzyl oder eine andere Aminoschutzgruppe bedeutet,
zu einer Verbindung der Formel I
worin
- R: 4-R¹-Piperazinyl,
- R': 2-R²-5-R³-Pyrrol-1-yl-carbonyl, 4-R⁴-Piperazin-1-yl-carbonyl, 1,4-Dihydro-benzo[*d*][1,2]oxazin-3-yl-carbonyl oder 3,4-Dihydro-benzo-1*H*-phthalazin-2-yl-carbonyl,
- R¹: Benzyl oder eine andere Aminoschutzgruppe,
- R⁴: H, Benzyl oder eine andere Aminoschutzgruppe,
- R², R³: jeweils unabhängig voneinander H oder Alkyl mit 1-6 C-Atomen, bedeuten,
umsetzt,
daß man die so erhaltene Verbindung der Formel I
i) durch basische Verseifung zuerst in eine Verbindung der Formel VI und/oder sein Säureadditionssalz 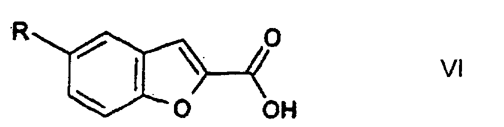 worin
   - R: 4-R¹-Piperazinyl und
   - R¹: Benzyl oder eine andere Aminoschutzgruppe bedeutet, umwandelt, und anschließend mit Ammoniak in eine Verbindung der Formel VII 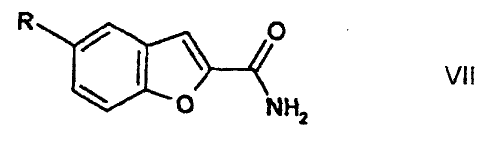 worin
   - R: 4-R¹-Piperazinyl und
   - R¹: Benzyl oder eine andere Aminoschutzgruppe bedeutet,
   umwandelt,
   oder
ii) direkt mit Ammoniak in eine Verbindung der Formel VII 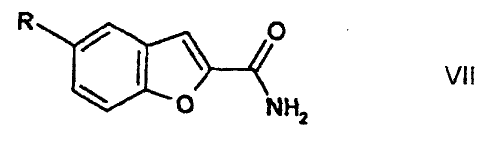
worin
- R: 4-R¹-Piperazinyl und
- R¹: Benzyl oder eine andere Aminoschutzgruppe bedeutet,
umwandelt,
daß man die so erhaltene Verbindung der Formel VII durch Abspaltung der Schutzgruppe R' in 5-(1-Piperazinyl)-benzofuran-2-carboxamid oder ein Säureadditionssalz umwandelt, und
daß man 5-(1-Piperazinyl)-benzofuran-2-carboxamid mit 3-(4-Chlorbutyl)-5-cyan-indol zu 1-[4-(5-Cyan-indol-3-yl)-butyl]-4-(2-carbamoyl-benzofuran-5-yl)-piperazin umsetzt und
gegebenenfalls in sein Säureadditionssalz überführt.

3-(4-Chlorbutyl)-5-cyan-indol ist bekannt aus DE 4101686, 1-[4-(5-Cyanindol-3-yl)-butyl]-4-(2-carbamoyl-benzofuran-5-yl)-piperazin ist bekannt aus DE 4333254.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation. Rf-Werte an Kieselgel.

### Beispiel 1

### (2,5-Dimethyl-pyrrol-1-yl)-5-brom-benzofuran-2-yl-methanon

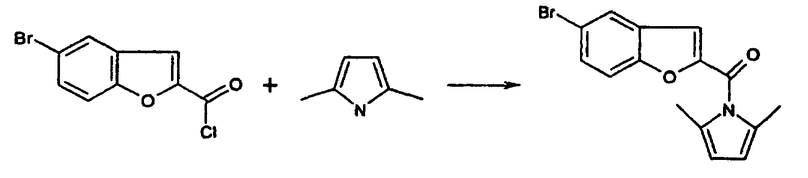

0,3 g Natriumhydrid (60%-Susp. in Paraffinöl) werden in 10 ml THF vorgelegt und 0,5 mL 2,5-Dimethyl-pyrrol in 10 ml THF (5 Minuten) zugetropft. Nach 1 Stunde Rühren bei 50 °C liegt eine rötliche Suspension vor. Bei 25°C wird 1,3 g 5-Brom-benzofuran-2-carbonylchlorid in 10 ml THF (5 Minuten) zugetropft und 2 Stunden nachgerührt. Es folgt die Zugabe von 100 ml VE-Wasser und 100 ml Ethylacetat.
Die abgetrennte organische Phase wurde noch 2 x mit 100 ml Wasser gewaschen und im Vakuum eingeengt. Das verbleibende Öl liefert nach Chromatografie über Kieselgel (Eluent Heptan/Ethylacetat 4:1) 700 mg gelbe Kristalle (Ausbeute 44%), Fp 115-116°.

### Beispiel 2

### 5-Brombenzofuran-2-carbonsäurediethylamid

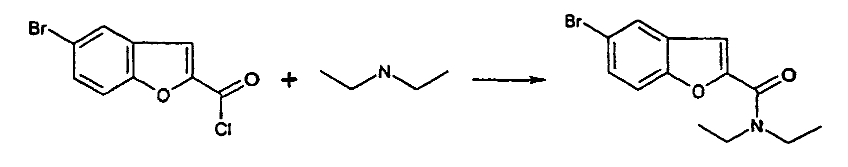

1,3 g 5-Brom-benzofuran-2-carbonylchlorid, 1 mL Ethyldiisopropylamin und 30 mL Toluol werden gemischt und unter Rühren zur braun gefärbten Lösung 0,62 mL Diethylamin zugegeben. Es bildet sich ein Niederschlag unter schwacher Exothermie. Nach 5 Minuten wurde mit 30 ml VE-Wasser versetzt und die Phasen getrennt. Die organische Phase wurde mit 1.) 20 ml 1 N HCl 2.) 20 ml 1 N NaOH 3.) 20 ml Wasser und anschließend mit 20 ml gesättigter NaCl-Lösung gewaschen und hierauf im Vakuum von Solvenzanteilen befreit. Das verbleibende, gelbliches Öl bildet nach Chromatographie an Kieselgel in Eluenz MtB-Ether/Heptan 2:1. Auswaage: 1,3 g farblose Kristalle. (Ausbeute: 88%), Fp .79-81°.

### Beispiel 3

### Di-tert-Butyl N-(5-brom-benzofuran-2-carbonyl-)imidodicarbonat

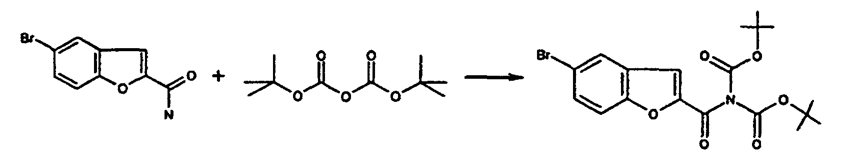

In 100 mL THF werden 12 g 5-Brombenzofuran-2-carboxamid, 23,5 mL BOC₂O, 600 mg DMAP und 8 mL Triethylamin bei 20°C eingetragen. In einer endothermen Reaktion bildet sich in 3 h eine klare, orange Lösung. Sie wird auf 25 °C erwärmt und mit 100 ml Wasser und 100 ml Ethylacetat versetzt. Die organische Phase wird abgetrennt, 2 mal mit 100 ml Wasser und 100 mL gesättigter Kochsalzlösung gewaschen. Die organische Phase wird eingeengt und bildet eine Mischung aus Öl und Kristallen (22 g/Ausbeute 40 %). Nach Kristallisation des Rohproduktes aus 160 ml Ethanol fallen 9,0 g gelbe Kristalle an, Fp. 138-139°.

### Beispiel 4

### 5-Brombenzofuran-2-carbonsäuredibenzylamid

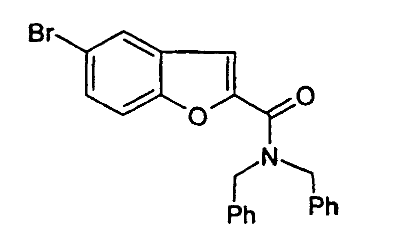

Zu 5,2 g 5-Brom-benzofuran-2-carbonsäurechlorid in 100 ml Toluol werden unter Rühren bei 50 -60 °C eine Lösung aus 50 ml Toluol und 7,9 g Dibenzylamin innerhalb von 10 min. zugetropft. Es fällt ein farbloser Feststoff an. Nach beendetem Zutropfen wird noch 3 Std. bei 100-110°C gerührt. Nach Abkühlen auf 10 °C wird das feste Produkt ( Dibenzylammoniumchlorid) abgesaugt. Danach versetzt man das Filtrat mit einer Mischung aus 150 ml Wasser und 10 g Natriumcarbonat und schüttelt gut durch. Die organische Phase wird abgetrennt, nochmals mit 100 ml Wasser gewaschen, mit Natriumsulfat getrocknet und filtriert. Das Filtrat engt man am Rotavapor im Vakuum bis zum Rückstand ein (Rückstand: 9,5 g ). Nach Umkristallisation aus 100 ml Methanol verbleiben nach Isolierung 6,5 g Produkt (Ausbeute 77%)
Fp. 114-115°.

### Beispiel 5

### 5-(4-Benzyl-piperazin-1-yl)-brombenzofuran-2-carbonsäuredibenzylamid

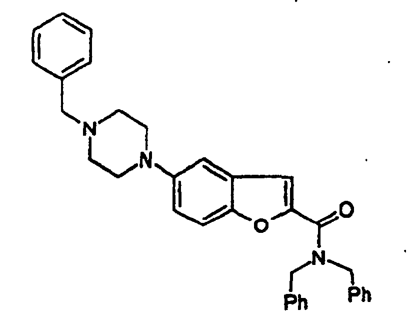

Unter Stickstoff wurden 0,08 mg Pd2DBA3 und 0,007 g 2-Dicyclohexylphosphino-2'-dimethylamino-biphenyl in 40 ml Toluol bei 25 °C für 20 min gerührt. Anschließend werden 1,58 g 5-Brom-2,3-dihydro-benzofuran-2-. carbonsäuredibenzylamid, 0,98 g 1-Benzylpiperazin und 1,43 g Natriumtert.-butylat zugesetzt und 2 Std. bei 120 °C gerührt. Die erkaltete Reaktionsmischung wird unter Rühren in eine Mischung aus 150 ml Wasser und 5 ml 37%iger Salzsäure eingerührt. Man neutralisiert das Reaktionsgemisch mit 1,5 g Natriumcarbonat und extrahiert die Phase 3 - mal mit 100 ml Ethylacetat. Die vereinigten organischen Phasen trocknet man mit 5 g Natriumsulfat und engt das Filtrat im Vakuum bis zum harzartigen Rückstand ein (1,8 g Rohprodukt). Das Rohprodukt wird in 100 ml Ethylacetat gelöst, mit Aktivkohle geklärt und filtriert. Durch Zugabe von 25 mL 2-molarer ethanolischer Salzsäure fällt man das Piperazin-Produkt als Hydrochlorid aus, filtriert, wäscht die Kristalle mit 10 mL Ethylacetat nach und trocknet im Vakuum bei 40 °C. Auswaage: 1,5 g / Ausbeute 53 %. Fp. 196 - 198 °.

## Patentansprüche

1. Benzofuranderivate der Formel I 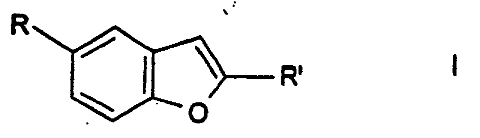 worin
R 1-Piperazinyl, 4-R¹-Piperazinyl oder L,
R' Z-R²-5-R³-Pyrrol-1-yl-carbonyl, 4-R⁴-Piperazin-1-yl-carbonyl, N,N-Di-(tert.-Butyloxycarbonyl)-aminocarbonyl, 1,4-Dihydro-benzo[*d*][1,2]oxazin-3-yl-carbonyl oder 3,4-Dihydro-benzo-1*H*-phthalazin-2-yl-carbonyl,
L Cl, Br, I oder eine freie OH-Gruppe, oder ein aktivierter Ester ein Imidazolid oder Alkylsulfonyloxy mit 1 bis 6 C-Atomen oder Arylsulfonyloxy mit 6 bis 10 C-Atomen,
R¹, R⁴ jeweils unabhängig voneinander H, Benzyl oder eine andere Aminoschutzgruppe,
R², R³ jeweils unabhängig voneinander H oder Alkyl mit 1-6 C-Atomen,
R⁵, R⁶ jeweils unabhängig voneinander Alkyl mit 1-6 C-Atomen,
Hal F, Cl, Br oder I
bedeuten,
sowie deren Salze.

2. Verbindungen nach Anspruch 1
a) (5-Brom-benzofuran-2-yl)-(2,5-dimethyl-pyrrol-1-yl)-methanon;
b) (4-Benzyl-piperazin-1-yl)-[5-(4-benzyl-piperazin-1-yl)-benzofuran-2-yl]-methanon;
c) [5-(4-Benryl-piperazin-1-yl)-benzofuran-2-yl]-(1,4-dihydrobenzo[d][1,2]oxazin-3-yl-methanon;
d) [5-(4-Benzyl-piperazin-1-yl)-benzofuran-2-yl]-(3,4-dihydro-1*H*phthalazin-2-yl)-methanon;
sowie deren Salze.

3. Verwendung der Verbindungen der Formel I nach Anspruch 1,
worin
R Cl, Br, I oder 4-R¹-Piperazinyl,
R' 2-R²-5-R³-Pyrrol-1-yl-carbonyl, 4-R⁴-Piperazin-1-yl-carbonyl, N,N-Di-(tert.-Butyloxycarbonyl)-aminocarbonyl, 1,4-Dihydro-benzo[*d*][1,2]oxazin-3-yl-carbonyl oder 3,4-Dihydro-benzo-1*H*-phthalazin-2-yl-carbonyl,
R¹ Benzyl oder eine andere Aminoschutzgruppe,
R⁴ H, Benzyl oder eine andere Aminoschutzgruppe,
R², R³ jeweils unabhängig voneinander H oder Alkyl mit 1-6 C-Atomen,
bedeuten,
bei der Synthese von 1-[4-(5-Cyan-indol-3-yl)-Butyl]-4-(2-carbamoyl-benzofuran-5-yl)-piperazin und dessen Salzen, **dadurch gekennzeichnet,**
**daß** man 3-R-6-hydroxy-benzaldehyd,
worin R Cl, Br oder I bedeutet,
mit einer Verbindung der Formel II
X-CH₂-CO-Q II
worin
X Cl, Br, I oder eine freie OH-Gruppe oder ein aktiverter Ester, ein Imidazolid oder Alkylsufonyloxy mit 1 bis 6 C-Atomen oder Arylsulfonyloxy mit 6 bis 10 C-Atomen,
Q OH oder OR" und
R" Alkyl mit 1-6 C-Atomen,
bedeutet,
zu einer Verbindung der Formel IV 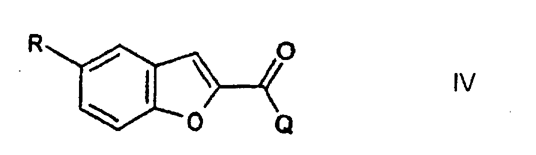 worin
R Cl, Br oder I,
und Q die angegebenen Bedeutungen bedeutet,
umsetzt,
**daß** man in der so erhaltenen Verbindung Q in Cl, Br, I oder einen aktiverten Ester, ein Imidazolid oder Alkylsufonyloxy mit 1 bis 6 C-Atomen oder Arylsulfonyloxy mit 6 bis 10 C-Atomen umwandelt,
**daß** man die so erhaltene Verbindung mit einer Verbindung der Formel III
R'-H III
worin
R' 2-R²-5-R³-Pyrrol-1-yl-carbonyl, 4-R⁴-Piperazin-1-yl-carbonyl, N,N-Di-(tert.-Butyloxycarbonyl)-aminocarbonyl, 1,4-Dihydrobenzo[*d*][1,2]oxazin-3-yl-carbonyl oder 3,4-Dihydro-benzo-1*H*-phthalazin-2-yl-carbonyl
bedeutet,
und R², R³ und R⁴ die angegebenen Bedeutungen haben,
zu einer Verbindung der Formel I
worin
R Cl, Br oder I,
R' 2-R²-5-R³-Pyrrol-1-yl-carbonyl, 4-R⁴-Piperazin-1-yl-carbonyl, N,N-Di-(tert.-Butyloxycarbonyl)-aminocarbonyl, 1,4-Dihydro-benzo[*d*][1,2]oxazin-3-yl-carbonyl oder 3,4-Dihydro-benzo-1*H*-phthalazin-2-yl-carbonyl,
R⁴ H, Benzyl oder eine andere Aminoschutzgruppe,
R², R³ jeweils unabhängig voneinander H oder Alkyl mit 1-6 C-Atomen,
bedeuten,
umsetzt,
**daß** man in der so erhaltenen Verbindung der Formel I den Rest R in einen anderen Rest R umwandelt,
indem man Übergangsmetall-katalysiert mit einer Verbindung der Formel V
4-R¹-Piperazin V
worin
R' Benzyl oder eine Aminoschutzgruppe bedeutet,
zu einer Verbindung der Formel I
worin
R 4-R¹-Piperazinyl,
R' 2-R²-5-R³-Pyrrol-1-yl-carbonyl, 4-R⁴-Piperazin-1-yl-carbonyl, N,N-Di-(tert.-Butyloxycarbonyl)-aminocarbonyl, 1,4-Dihydro-benzo[*d*][1,2]oxazin-3-yl-carbonyl oder 3,4-Dihydro-benzo-1*H*-phthalazin-2-yl-carbonyl,
R¹ Benzyl oder eine Aminoschutzgruppe,
R⁴ H, Benzyl oder eine andere Aminoschutzgruppe,
R², R³ jeweils unabhängig voneinander H oder Alkyl mit 1-6 C-Atomen,
bedeuten,
umsetzt,
**daß** man die so erhaltene Verbindung der Formel I
i) durch basische Verseifung zuerst in eine Verbindung der Formel VI und/oder sein Säureadditionssalz 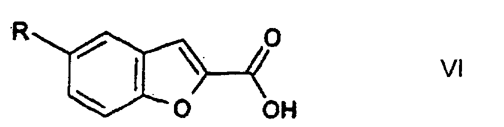 worin
R 4-R¹-Piperazinyl und
R¹ Benzyl oder eine andere Aminoschutzgruppe
bedeutet,
umwandelt, und anschließend mit Ammoniak in eine Verbindung der Formel VII 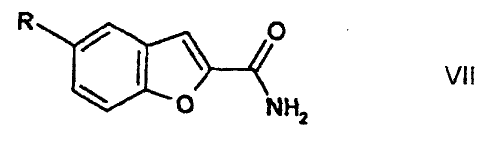 worin
R 4-R¹-Piperazinyl und
R¹ Benzyl oder eine andere Aminoschutzgruppe bedeutet,
umwandelt,
oder
ii) direkt mit Ammoniak in eine Verbindung der Formel VII 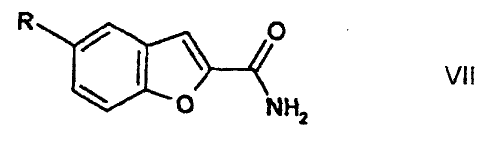 worin
R 4-R¹-Piperazinyl und
R¹ Benzyl oder eine andere Aminoschutzgruppe
bedeutet,
umwandelt,
**daß** man die so erhaltene Verbindung der Formel VII durch Abspaltung der Schutzgruppe R' in 5-(1-Piperazinyl)-benzofuran-2-carboxamid oder ein Säureadditionssalz umwandelt, und daß man 5-(1-Piperazinyl)-benzofuran-2-carboxamid mit 3-(4-Chlorbutyl)-5-cyan-indol zu 1-[4-(5-Cyan-indol-3-yl)-butyl]-4-(2-carbamoyl-benzofuran-5-yl)-piperazin umsetzt und gegebenenfalls in sein Säureadditionssalz überführt.

4. Verwendung der Verbindungen der Formel I nach Anspruch 1 als Zwischenprodukte zur Synthese von Arzneimitteln.

5. Verwendung der Verbindungen der Formel I nach Anspruch 1 als Zwischenprodukte zur Synthese von Arzneimitteln, die Wirkungen auf das Zentralnervensystem zeigen.

## Claims

1. Benzofuran derivatives of the formula I 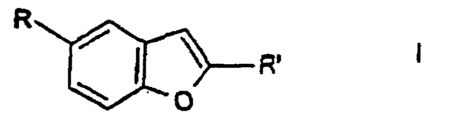 in which
R denotes 1-piperazinyl, 4-R¹-piperazinyl or L,
R' denotes 2-R²-5-R³-pyrrol-1-ylcarbonyl, 4-R⁴-piperazin-1-ylcarbonyl, N,N-di(tert-butoxycarbonyl)aminocarbonyl, 1,4-dihydrobenzo[*d*]-1,2-oxazin-3-ylcarbonyl or 3,4-dihydrobenzo-1*H*-phthalazin-2-ylcarbonyl,
L denotes Cl, Br, I or a free OH group, or an activated ester, an imidazolide or alkylsulfonyloxy having 1 to 6 C atoms or arylsulfonyloxy having 6 to 10 C atoms,
R¹, R⁴ each, independently of one another, denote H, benzyl or another amino-protecting group,
R², R³ each, independently of one another, denote H or alkyl having 1-6 C atoms,
R⁵, R⁶ each, independently of one another, denote alkyl having 1-6 C atoms,
Hal denotes F, Cl, Br or I,
and salts thereof.

2. Compounds according to Claim 1
a) (5-bromobenzofuran-2-yl)(2,5-dimethylpyrrol-1-yl)methanone;
b) (4-benzylpiperazin-1-yl)[5-(4-benzylpiperazin-1-yl)benzofuran-2-yl]-methanone;
c) [5-(4-benzylpiperazin-1-yl)benzofuran-2-yl](1,4-dihydrobenzo[*d*]-1,2-oxazin-3-ylmethanone;
d) [5-(4-benzylpiperazin-1-yl)benzofuran-2-yl](3,4-dihydro-1*H*-phthalazin-2-yl)methanone;
and salts thereof.

3. Use of the compounds of the formula I according to Claim 1 in which
R denotes Cl, Br, I or 4-R¹-piperazinyl,
R' denotes 2-R²-5-R³-pyrrol-1-ylcarbonyl, 4-R⁴-piperazin-1-ylcarbonyl, N,N-di(tert-butoxycarbonyl)aminocarbonyl, 1,4-dihydrobenzo[*d*]-1,2-oxazin-3-ylcarbonyl or 3,4-dihydrobenzo-1*H*-phthalazin-2-ylcarbonyl,
R¹ denotes benzyl or another amino-protecting group,
R⁴ denotes H, benzyl or another amino-protecting group,
R², R³ each, independently of one another, denote H or alkyl having 1-6 C atoms,
in the synthesis of
1-[4-(5-cyanoindol-3-yl)butyl]-4-(2-carbamoylbenzofuran-5-yl)piperazine and salts thereof, **characterised in that**
3-R-6-hydroxybenzaldehyde,
in which R denotes Cl, Br or I,
is reacted with a compound of the formula II
X-CH₂-CO-Q II
in which
X denotes Cl, Br, I or a free OH group or an activated ester, an imidazolide or alkylsulfonyloxy having 1 to 6 C atoms or arylsulfonyloxy having 6 to 10 C atoms,
Q denotes OH or OR" and
R" denotes alkyl having 1-6 C atoms,
to give a compound of the formula IV 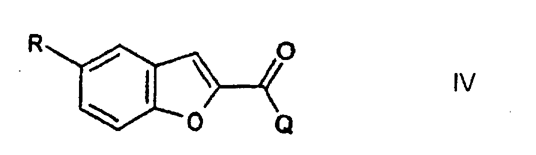 in which
R denotes Cl, Br or I,
and Q has the meanings indicated,
**in that** Q in the compound obtained in this way is converted into Cl, Br, I or an activated ester, an imidazolide or alkylsulfonyloxy having 1 to 6 C atoms or arylsulfonyloxy having 6 to 10 C atoms,
**in that** the compound obtained in this way is reacted with a compound of the formula III
R'-H III
in which
R' denotes 2-R²-5-R³-pyrrol-1-ylcarbonyl, 4-R⁴-piperazin-1-yl-carbonyl, N,N-di(tert-butoxycarbonyl)aminocarbonyl, 1,4-dihydrobenzo[*d*]-1,2-oxazin-3-ylcarbonyl or 3,4-dihydrobenzo-1H-phthalazin-2-ylcarbonyl,
and R², R³ and R⁴ have the meanings indicated,
to give a compound of the formula I
in which
R denotes Cl, Br or I,
R' denotes 2-R²-5-R³-pyrrol-1-ylcarbonyl, 4-R⁴-piperazin-1-ylcarbonyl, N,N-di(tert-butoxycarbonyl)aminocarbonyl, 1,4-dihydrobenzo[*d*]-1,2-oxazin-3-ylcarbonyl or 3,4-dihydrobenzo-1*H*-phthalazin-2-ylcarbonyl,
R⁴ denotes H, benzyl or another amino-protecting group,
R², R³ each, independently of one another, denote H or alkyl having 1-6 C atoms,
**in that** the radical R in the compound of the formula I obtained in this way is converted into another radical R
by reaction, with transition-metal catalysis, with a compound of the formula V
4-R¹-piperazine V
in which
R¹ denotes benzyl or an amino-protecting group,
to give a compound of the formula I
in which
R denotes 4-R¹-piperazinyl,
R' denotes 2-R²-5-R³-pyrrol-1-ylcarbonyl, 4-R⁴-piperazin-1-ylcarbonyl, N,N-di(tert-butoxycarbonyl)aminocarbonyl, 1,4-dihydrobenzo[*d*]-1,2-oxazin-3-ylcarbonyl or 3,4-dihydrobenzo-1*H*-phthalazin-2-ylcarbonyl,
R¹ denotes benzyl or an amino-protecting group,
R⁴ denotes H, benzyl or another amino-protecting group,
R², R³ each, independently of one another, denote H or alkyl having 1-6 C atoms,
**in that** the compound of the formula I obtained in this way
i) is firstly converted by basic saponification into a compound of the formula VI and/or an acid-addition salt thereof 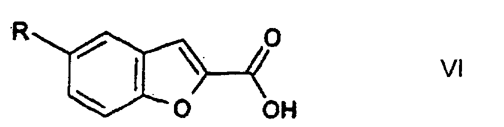 in which
R denotes 4-R¹-piperazinyl and
R¹ denotes benzyl or another amino-protecting group,
and is subsequently converted using ammonia into a compound of the formula VII 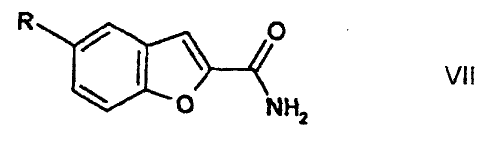 in which
R denotes 4-R¹-piperazinyl and
R¹ denotes benzyl or another amino-protecting group,
or
ii) is converted directly using ammonia into a compound of the formula VII 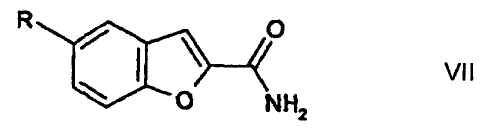 in which
R denotes 4-R¹-piperazinyl and
R¹ denotes benzyl or another amino-protecting group,
**in that** the compound of the formula VII obtained in this way is converted by removal of the protecting group R¹ into 5-(1-piperazinyl)-benzofuran-2-carboxamide or an acid-addition salt, and
**in that** 5-(1-piperazinyl)benzofuran-2-carboxamide is reacted with 3-(4-chlorobutyl)-5-cyanoindole to give 1-[4-(5-cyanoindol-3-yl)butyl]-4-(2-carbamoylbenzofuran-5-yl)piperazine and is optionally converted into an acid-addition salt thereof.

4. Use of the compounds of the formula I according to Claim 1 as intermediates for the synthesis of medicaments.

5. Use of the compounds of the formula I according to Claim 1 as intermediates for the synthesis of medicaments which exhibit actions on the central nervous system.

## Revendications

1. Dérivés de benzofurane de la formule I 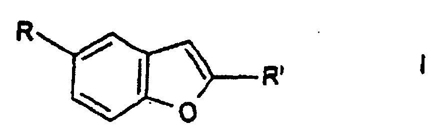 dans laquelle
R représente 1-pipérazinyle, 4-R¹-pipérazinyle ou L,
R' représente 2-R²-5-R³-pyrrol-1-ylcarbonyle, 4-R⁴-pipérazine-1-ylcarbonyle, N,N-di(tert-butoxycarbonyl)aminocarbonyle, 1,4-dihydrobenzo[*d*]-1,2-oxazine-3-yl-carbonyle ou 3,4-dihydrobenzo-1*H*-phthalazine-2-yl-carbonyle,
L représente Cl, Br, I ou, un groupe OH libre, ou un ester activé, un imidazolide ou alkylsulfonyloxy ayant 1 à 6 atomes de C ou arylsulfonyloxy ayant 6 à 10 atomes de C,
R¹, R⁴ chacun, indépendamment l'un de l'autre, représente H, benzyle ou un autre groupe de protection amino,
R², R³ chacun, indépendamment l'un de l'autre, représente H ou alkyle ayant 1-6 atomes de C,
R⁵, R⁶ chacun, indépendamment l'un de l'autre, représente alkyle ayant 1-6 atomes de C,
Hal représente F, CI, Br ou I,
et des sels afférents.

2. Composés selon la revendication 1
a) (5-bromobenzofurane-2-yl)(2,5-diméthylpyrrol-1-yl)méthanone;
b) (4-benzylpipérazine-1-yl)[5-(4-benzylpipérazine-1-yl)benzofurane-2-yl]méthanone;
c) [5-(4-benzylpipérazine-1-yl)benzofurane-2-yl](1,4-dihydrobenzo[*d*]-1,2-oxazine-3-ylméthanone;
d) [5-(4-benzylpipérazine-1-yl)benzofurane-2-yl](3,4-dihydro-1*H*-phthalazine-2-yl)méthanone;
et des sels afférents.

3. Utilisation des composés de la formule I selon la revendication 1,
dans laquelle
R représente Cl, Br, I ou 4-R¹-pipérazinyle,
R' représente 2-R²-5-R³-pyrrol-1-ylcarbonyle, 4-R⁴-pipérazine-1-ylcarbonyle, N,N-di(tert-butoxycarbonyl)aminocarbonyle, 1,4-dihydrobenzo[*d*]-1,2-oxazine-3-yl-carbonyle ou 3,4-dihydrobenzo-1*H*-phthalazine-2-yl-carbonyle,
R' représente benzyle ou un autre groupe de protection amino,
R⁴ représente H, benzyle ou un autre groupe de protection amino,
R², R³ chacun, indépendamment l'un de l'autre, représente H ou alkyle ayant 1-6 atomes de C,
dans la synthèse de
1-[4-(5-cyanoindole-3-yl)butyl]-4-(2-carbamoylbenzofurane-5-yl)-pipérazine et des sels afférents, **caractérisée en ce que** 3-R-6-hydroxybenzaldéhyde,
dans lequel R représente CI, Br ou I,
est amené à réagir avec un composé de la formule II
X-CH₂-CO-Q II
dans laquelle
X représente Cl, Br, I ou un groupe OH libre ou un ester activé, un imidazolide ou alkylsulfonyloxy ayant 1 à 6 atomes de C ou arylsulfonyloxy ayant 6 à 10 atomes de C,
Q représente OH ou OR" et
R" représente alkyle ayant 1-6 atomes de C,
pour donner un composé de la formule IV 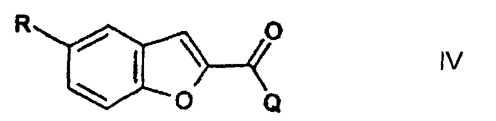 dans laquelle
R représente Cl, Br ou I,
et Q a les significations indiquées,
**en ce que** Q dans le composé obtenu de cette façon est converti en Cl, Br, I ou un ester activé, un imidazolide ou alkylsulfonyloxy ayant 1 à 6 atomes de C ou arylsulfonyloxy ayant 6 à 10 atomes de C,
**en ce que** le composé obtenu de cette façon est amené à réagir avec un composé de la formule III
R'-H III
dans laquelle
R' représente 2-R²-5-R³-pyrrol-1-ylcarbonyle, 4-R⁴-pipérazine-1-ylcarbonyle, N,N-di(tertbutoxycarbonyl)aminocarbonyle, 1,4-dihydrobenzo[*d*]-1,2-oxazine-3-ylcarbonyle ou 3,4-dihydrobenzo-1*H*-phthalazine-2-ylcarbonyle,
et R², R³ et R⁴ ont les significations indiquées,
pour donner un composé de la formule I
dans laquelle
R représente CI, Br ou I,
R' représente 2-R²-5-R³-pyrrol-1-ylcarbonyle, 4-R⁴-pipérazine-1-ylcarbonyle, N,N-di(tert-butoxycarbonyl)aminocarbonyle, 1,4-dihydrobenzo[*d*]-1,2-oxazine-3-yl-carbonyle ou 3,4-dihydrobenzo-1*H*-phthalazine-2-yl-carbonyle,
R⁴ représente H, benzyle ou un autre groupe de protection amino,
R², R³ chacun, indépendamment l'un de l'autre, représente H ou alkyle ayant 1-6 atomes de C,
**en ce que** le radical R dans le composé de la formule I obtenu de cette façon est converti selon un autre radical R par réaction, avec une catalyse de métal de transition, avec un composé de la formule V
4-R¹-pipérazine V
dans laquelle
R' représente benzyle ou un groupe de protection amino,
pour donner un composé de la formule I
dans laquelle
R représente 4-R¹-pipérazinyle,
R' représente 2-R²-5-R³-pyrrol-1-ylcarbonyle, 4-R⁴-pipérazine-1-ylcarbonyle, N,N-di(tert-butoxycarbonyl)aminocarbonyle, 1,4-dihydrobenzo[*d*]-1,2-oxazine-3-yl-carbonyle ou 3,4-dihydrobenzo-1*H*-phthalazine-2-yl-carbonyle,
R' représente benzyle ou un groupe de protection amino,
R⁴ représente H, benzyle ou un autre groupe de protection amino,
R², R³ chacun, indépendamment l'un de l'autre, représente H ou alkyle ayant 1-6 atomes de C,
**en ce que** le composé de la formule I obtenu de cette façon
i) est tout d'abord converti par saponification basique selon un composé de la formule VI et/ou un sel par addition d'acide afférent 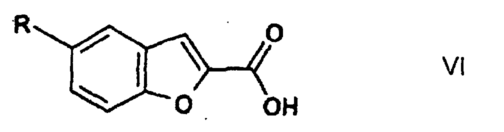 dans laquelle
R représente 4-R¹-pipérazinyle et
R¹ représente benzyle ou un autre groupe de protection amino,
et est ensuite converti en utilisant de l'ammoniaque selon un composé de la formule VII 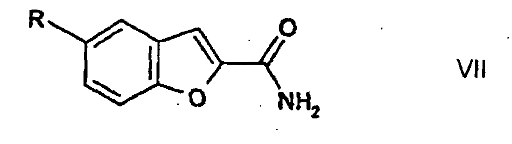 dans laquelle
R représente 4-R¹-pipérazinyle et
R' représente benzyle ou un autre groupe de protection amino,
ou
ii) est converti directement en utilisant de l'ammoniaque selon un composé de la formule VII 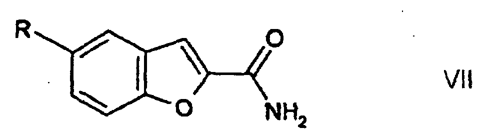 dans laquelle
R représente 4-R¹-pipérazinyle et
R¹ représente benzyle ou un autre groupe de protection amino,
**en ce que** le composé de la formule VII obtenu de cette façon est converti par enlèvement du groupe de protection R' selon 5-(1-pipérazinyl)benzofurane-2-carboxamide ou un sel par addition d'acide, et
**en ce que** 5-(1-pipérazinyl)benzofurane-2-carboxamide est amené à réagir avec 3-(4-chlorobutyl)-5-cyanoindole pour donner 1-[4-(5-cyanoindol-3-yl)butyl]-4-(2-carbamoylbenzofurane-5-yl)pipérazine et est optionnellement converti selon un sel par addition d'acide afférent.

4. Utilisation des composés de la formule I selon la revendication 1 en tant qu'intermédiaires pour la synthèse de médicaments.

5. Utilisation des composés de la formule I selon la revendication 1 en tant qu'intermédiaires pour la synthèse de médicaments qui présentent des actions sur le système nerveux central.
